# EUROPEAN PATENT APPLICATION

(11) **EP 4 755 383 A1**
(43) Date of publication of application: **10.06.2026**
(21) Application number: 24848262.2
(22) Date of filing: 30.07.2024
(51) Int. Cl.: A61K 31/4355, A61P 35/00

(54) **USE OF PYRIDONE CARBOXAMIDE COMPOUND IN TREATMENT OF DISEASES ASSOCIATED WITH NEUROFIBROMATOSIS TYPE I**

(30) Priority: 31.07.2023 CN 202310948704
(71) Applicant: Chia Tai Tianqing Pharmaceutical Group Co., Ltd., Lianyungang, Jiangsu 222062 (CN); Shouyao Holdings (Beijing) Co., Ltd., Beijing 100176 (CN)
(72) Inventor: WAN, Xiaojing, Lianyungang, Jiangsu 222062 (CN); ZHANG, Xiquan, Lianyungang, Jiangsu 222062 (CN); ZHAO, Wei, Lianyungang, Jiangsu 222062 (CN); WANG, Xunqiang, Lianyungang, Jiangsu 222062 (CN); YU, Ding, Lianyungang, Jiangsu 222062 (CN)
(74) Representative: Grünecker Patent- und Rechtsanwälte PartG mbB
(86) International application number: PCT/CN2024/108441
(87) International publication number: WO 2025/026307

(57) **Abstract**

The present invention relates to a use of a pyridone carboxamide compound in the treatment of diseases associated with neurofibromatosis type I, and in particular to a use of 6-(2-chloro-4-iodophenylamino)-N-(2-hydroxyethoxy)-5-methyl-4-oxo-4,5-dihydrofuro[3,2-c]pyridine-7-carboxamide or a pharmaceutically acceptable salt thereof in the treatment of diseases associated with neurofibromatosis type I.

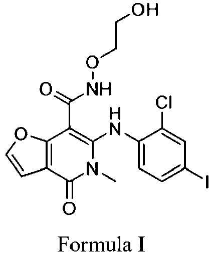

## Description

### CROSS-REFERENCE TO RELATED APPLICATIONS

The present application claims priority and benefit to the Chinese Patent Application No. 202310948704.0 filed with China National Intellectual Property Administration on July 31, 2023, the disclosure of which is incorporated herein by reference in its entirety.

### TECHNICAL FIELD

The present application belongs to the field of pharmaceutics and relates to pharmaceutical use of a pyridone carboxamide compound. Specifically, the present application relates to use of 6-(2-chloro-4-iodophenylamino)-*N*-(2-hydroxyethoxy)-5-methyl-4-oxo-4,5-dihydrofuro[3,2-c]pyridine-7-carboxamide or a pharmaceutically acceptable salt thereof in the treatment of diseases associated with neurofibromatosis type I.

### BACKGROUND

Cellular signal transduction pathways play an important role in cell growth, proliferation, and differentiation. The Ras/Raf/MEK/ERK pathway is a major signal transduction pathway, and it transmits signals from multiple cell surface receptors to transcription factors that regulate gene expression within the nucleus. It has been found that abnormal activation of the Ras/Raf/MEK/ERK pathway is often observed in malignantly transformed cells, and inhibiting this pathway is considered beneficial for treating hyperproliferative diseases. Due to its position downstream of Ras and Raf, MEK is a key member of this pathway and a prominent therapeutic target. Neurofibromatosis type I is an autosomal dominant genetic neoplastic disease caused by mutations in the *NF1* gene, which encodes neurofibromin. *NF1* gene mutations can lead to dysregulation of the RAS/RAF/MEK/ERK signaling pathway, thereby contributing to tumor formation and other related diseases.

WO2012059041A1 discloses a 6-arylamino pyridone carboxamide compound having a chemical structure represented by formula I, which exhibits MEK inhibitory activity and can be used for treating inflammatory diseases, cancers, and other hyperproliferative diseases.

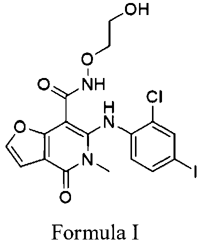

### SUMMARY

In one aspect, the present application provides use of a compound of formula I or a pharmaceutically acceptable salt thereof for preparing a medicament for treating a disease associated with neurofibromatosis type I,

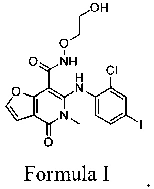

In another aspect, the present application also provides a method for treating a disease associated with neurofibromatosis type I, which comprises administering to a patient in need of the treatment an effective amount of the compound of formula I or the pharmaceutically acceptable salt thereof.

In another aspect, the present application also provides use of the compound of formula I or the pharmaceutically acceptable salt thereof for treating a disease associated with neurofibromatosis type I.

In another aspect, the present application also provides the compound of formula I or the pharmaceutically acceptable salt thereof for use in treating a disease associated with neurofibromatosis type I.

In yet another aspect, the present application also provides a pharmaceutical composition for use in treating a disease associated with neurofibromatosis type I, and the pharmaceutical composition comprises the compound of formula I or the pharmaceutically acceptable salt thereof.

In yet another aspect, the present application also provides use of the pharmaceutical composition of the compound of formula I or the pharmaceutically acceptable salt thereof for preparing a medicament for treating a disease associated with neurofibromatosis type I.

In yet another aspect, the present application also provides a method for treating a disease associated with neurofibromatosis type I, which comprises administering to a patient in need of the treatment an effective amount of the pharmaceutical composition of the compound of formula I or the pharmaceutically acceptable salt thereof. In yet another aspect, the present application also provides use of the pharmaceutical composition of the compound of formula I or the pharmaceutically acceptable salt thereof for treating a disease associated with neurofibromatosis type I.

In further another aspect, the present application provides a kit for use in treating a disease associated with neurofibromatosis type I, and the kit comprises the pharmaceutical composition of the compound of formula I or the pharmaceutically acceptable salt thereof and an instruction for use of the compound of formula I or the pharmaceutically acceptable salt thereof for treating the disease associated with neurofibromatosis type I.

### Compound of Formula I or Pharmaceutically Acceptable Salt Thereof, or Pharmaceutical Composition Thereof

The compound of formula I of the present application may be administered in its free base form or in its pharmaceutically acceptable salt form.

In some embodiments, the compound of formula I or the pharmaceutically acceptable salt thereof is in a crystalline form.

In some specific embodiments, the compound of formula I is in a crystalline form, which has diffraction peaks in an X-ray powder diffraction pattern at 2^{θ} values of 7.86°, 19.09°, 21.80°, 23.87°, 26.00°, and 28.12°. Reference can be made to WO2016019867 for the preparation method for and the related properties of the crystalline form of the compound of formula I.

The weight or dose of the compound of formula I or the pharmaceutically acceptable salt thereof referred to in the present application is based on the molecular weight of the compound of formula I, unless otherwise stated. In some embodiments, the compound of formula I or the pharmaceutically acceptable salt thereof, or the pharmaceutical composition thereof is the compound of formula I or the pharmaceutically acceptable salt thereof, or the pharmaceutical composition thereof at 0.1 mg-1000.0 mg, 0.5 mg-500.0 mg, 2 mg-250.0 mg, 5 mg-100 mg, or 5 mg-50 mg, preferably the compound of formula I or the pharmaceutically acceptable salt thereof, or the pharmaceutical composition thereof at 0.1 mg, 0.5 mg, 1.0 mg, 2.0 mg, 3.0 mg, 4.0 mg, 5.0 mg, 6.0 mg, 7.0 mg, 8.0 mg, 9.0 mg, 10.0 mg, 15.0 mg, 20.0 mg, 25.0 mg, 30.0 mg, 35.0 mg, 40.0 mg, 45.0 mg, 50.0 mg, 60.0 mg, 70.0 mg, 80.0 mg, 90.0 mg, 100.0 mg, 125.0 mg, 150.0 mg, 175.0 mg, 200.0 mg, 250.0 mg, or a range formed by any of the above values. In some embodiments, the compound of formula I or the pharmaceutically acceptable salt thereof, or the pharmaceutical composition thereof is the compound of formula I or the pharmaceutically acceptable salt thereof, or the pharmaceutical composition thereof at 5 mg, 10 mg, 15 mg, 50 mg, or 100 mg.

In some embodiments, the compound of formula I or the pharmaceutically acceptable salt thereof, or the pharmaceutical composition thereof is the pharmaceutical composition in a single dose of 0.1 mg-1000.0 mg, 0.5 mg-500.0 mg, 2.0 mg-250.0 mg, 5.0 mg-100.0 mg, or 5.0 mg-50.0 mg, preferably the compound of formula I or the pharmaceutically acceptable salt thereof, or the pharmaceutical composition thereof in a single dose of 0.1 mg, 0.5 mg, 1.0 mg, 2.0 mg, 3.0 mg, 4.0 mg, 5.0 mg, 6.0 mg, 7.0 mg, 8.0 mg, 9.0 mg, 10.0 mg, 15.0 mg, 20.0 mg, 25.0 mg, 30.0 mg, 35.0 mg, 40.0 mg, 45.0 mg, 50.0 mg, 60.0 mg, 70.0 mg, 80.0 mg, 90.0 mg, 100.0 mg, 125.0 mg, 150.0 mg, 175.0 mg, 200.0 mg, 250.0 mg, or a range formed by any of the above values.

In some embodiments, the compound of formula I or the pharmaceutically acceptable salt thereof, or the pharmaceutical composition thereof is the compound of formula I or the pharmaceutically acceptable salt thereof, or the pharmaceutical composition thereof in a single dose of 5 mg, 10 mg, or 50 mg.

In some embodiments, the pharmaceutical composition of the compound of formula I or the pharmaceutically acceptable salt thereof further comprises a pharmaceutically acceptable excipient.

In some embodiments, the pharmaceutical composition of the compound of formula I or the pharmaceutically acceptable salt thereof is selected from the group consisting of a granule, a tablet, a pill, and a capsule, preferably selected from the group consisting of a tablet and a capsule, and more preferably selected from a capsule.

In some embodiments, the pharmaceutical composition of the compound of formula I or the pharmaceutically acceptable salt thereof may be a capsule, which comprises the compound of formula I or the pharmaceutically acceptable salt thereof, a binder, a surface stabilizer, and a dispersant. In some embodiments, the pharmaceutical composition of the compound of formula I or the pharmaceutically acceptable salt thereof may be a capsule of the compound of formula I, which comprises the compound of formula I, hydroxypropylcellulose, sodium dodecyl sulfate, and sucrose. In some embodiments, the pharmaceutical composition of the compound of formula I or the pharmaceutically acceptable salt thereof further comprises an inert core, and the inert core is selected from the group consisting of a microcrystalline cellulose core, a sugar starch core, and a lactose core.

### Method of Administration

While the present disclosure exemplifies certain dosages and administration regimens, these examples are in no way limiting to the dosages and administration regimens that can be provided to a patient in practicing the present disclosure.

In some embodiments, the administration of the compound of formula I or the pharmaceutically acceptable salt thereof, or the pharmaceutical composition thereof follows a 28-day (4-week) administration cycle.

In some embodiments, the treatment cycle of the compound of formula I or the pharmaceutically acceptable salt thereof, or the pharmaceutical composition thereof involves repeating the administration cycle described above until the subject no longer derives benefit, experiences disease progression, or develops intolerable toxicity. In some embodiments, the treatment cycle of the compound of formula I or the pharmaceutically acceptable salt thereof, or the pharmaceutical composition thereof is 4-150, 4-90, or 8-60 administration cycles. In some embodiments, the treatment cycle of the compound of formula I or the pharmaceutically acceptable salt thereof, or the pharmaceutical composition thereof is 4-10 or 8-20 administration cycles.

In some embodiments, the compound of formula I or the pharmaceutically acceptable salt thereof, or the pharmaceutical composition thereof may be administered at a frequency of thrice every day, twice every day, once every day, once every two days, once every three days, once every four days, once every five days, once every six days, thrice every week, twice every week, once every week, once every two weeks, or once every three weeks. In some embodiments, the compound of formula I or the pharmaceutically acceptable salt thereof, or the pharmaceutical composition thereof is administered at a frequency of twice every day or once every day.

In some embodiments, the daily dose of the compound of formula I or the pharmaceutically acceptable salt thereof, or the pharmaceutical composition thereof is 0.1 mg-1000.0 mg, preferably 0.5 mg-500.0 mg, and more preferably 5.0 mg-200.0 mg, 5.0 mg-100.0 mg, 5.0 mg-70.0 mg, or 5.0 mg-50.0 mg. In some embodiments, the daily dose of the compound of formula I or the pharmaceutically acceptable salt thereof, or the pharmaceutical composition thereof is 0.5 mg, 1.0 mg, 2.0 mg, 3.0 mg, 4.0 mg, 5.0 mg, 6.0 mg, 7.0 mg, 8.0 mg, 9.0 mg, 10.0 mg, 15.0 mg, 20.0 mg, 25.0 mg, 30.0 mg, 35.0 mg, 40.0 mg, 45.0 mg, 50.0 mg, 60.0 mg, 70.0 mg, 80.0 mg, 90.0 mg, 100.0 mg, 125.0 mg, 150.0 mg, 175.0 mg, 200.0 mg, 250.0 mg, 300.0 mg, 400.0 mg, 500.0 mg, or a range formed by any of the above values. In some embodiments, the daily dose of the compound of formula I or the pharmaceutically acceptable salt thereof, or the pharmaceutical composition thereof is 5.0 mg, 15.0 mg, 50.0 mg, 70.0 mg, 100.0 mg, 150.0 mg, 175.0 mg, 200.0 mg, or a range formed by any of the above values. In some embodiments, the daily dose of the compound of formula I or the pharmaceutically acceptable salt thereof, or the pharmaceutical composition thereof is 5.0 mg, 15.0 mg, 50.0 mg, 70.0 mg, 100.0 mg, or a range formed by any of the above values.

In some embodiments, the compound of formula I or the pharmaceutically acceptable salt thereof, or the pharmaceutical composition thereof is administered in a single dose or in multiple doses.

In some embodiments, the compound of formula I or the pharmaceutically acceptable salt thereof, or the pharmaceutical composition thereof can be administered via multiple routes including, but not limited to, oral administration. In some embodiments, the compound of formula I or the pharmaceutically acceptable salt thereof, or the pharmaceutical composition thereof is administered orally under fasting conditions.

In some embodiments, the compound of formula I or the pharmaceutically acceptable salt thereof, or the pharmaceutical composition thereof is used as a single active agent.

### Disease Associated with Neurofibromatosis Type I

In some embodiments, the disease associated with neurofibromatosis type I is selected from the group consisting of neurofibromatosis type I and malignant peripheral nerve sheath tumor.

In some embodiments, the disease associated with neurofibromatosis type I is selected from the group consisting of symptomatic and inoperable neurofibromatosis type I and malignant peripheral nerve sheath tumor.

In some embodiments, the patient with the disease associated with neurofibromatosis type I is an adult patient. In some embodiments, the disease associated with neurofibromatosis type I is selected from the group consisting of neurofibromatosis type I and malignant peripheral nerve sheath tumor that cannot be completely surgically resected, require systemic therapy, and have measurable lesions.

In some embodiments, the disease associated with neurofibromatosis type I is neurofibromatosis type I.

In some embodiments, the neurofibromatosis type I is selected from the group consisting of benign neurofibromatosis type I and malignant neurofibromatosis type I.

In some embodiments, the neurofibromatosis type I is selected from the group consisting of central, visceral, cutaneous, and peripheral neurofibromatosis type I.

In some embodiments, the neurofibromatosis type I is selected from the group consisting of cutaneous neurofibroma, nodular neurofibroma, and plexiform neurofibroma.

In some embodiments, the neurofibromatosis type I is selected from the group consisting of multiple cutaneous neurofibroma and plexiform neurofibroma.

In some embodiments, the disease associated with neurofibromatosis type I is malignant peripheral nerve sheath tumor. In some embodiments, the malignant peripheral nerve sheath tumor arises from the malignant transformation of plexiform neurofibroma.

In some embodiments, the disease associated with neurofibromatosis type I is selected from the group consisting of cutaneous neurofibroma, plexiform neurofibroma, and peripheral nerve sheath tumor. In some embodiments, the disease associated with neurofibromatosis type I is selected from the group consisting of cutaneous neurofibroma, benign plexiform neurofibroma, and malignant peripheral nerve sheath tumor.

In some embodiments, the disease associated with neurofibromatosis type I is selected from plexiform neurofibroma that cannot be completely surgically resected. In some embodiments, the disease associated with neurofibromatosis type I is selected from symptomatic and inoperable plexiform neurofibroma (PN).

In some embodiments, the disease associated with neurofibromatosis type I is selected from the group consisting of cutaneous café-au-lait macules, axillary or inguinal freckling, optic nerve glioma, Lisch nodules (iris hamartoma), characteristic bony lesion (sphenoid dysplasia or long bone cortical dysplasia/thinning), plexiform neurofibroma, and malignant peripheral nerve sheath tumor.

In some embodiments, the patient with neurofibromatosis type I has inoperable symptomatic and/or progressive plexiform neurofibroma.

In some embodiments, the patient with neurofibromatosis type I meets at least 2 of the following criteria: a) 6 or more café-au-lait macules with a diameter (greatest dimension) greater than 5 mm before puberty, or a diameter greater than 15 mm after puberty; b) axillary or inguinal freckling; c) 2 or more neurofibromas of any type, or 1 plexiform neurofibroma; d) optic nerve glioma; e) 2 or more Lisch nodules (iris hamartomas); f) characteristic bony lesion, such as sphenoid dysplasia, or long bone cortical thickening with or without pseudarthrosis; g) having a first-degree relative (parent, sibling, or child) diagnosed with neurofibromatosis type I.

In some embodiments, the patient with neurofibromatosis type I meets at least 2 of the following criteria: a) 6 or more café-au-lait macules with a diameter (greatest dimension) greater than 5 mm before puberty, or a diameter greater than 15 mm after puberty; b) axillary or inguinal freckling; c) 2 or more neurofibromas of any type, or 1 plexiform neurofibroma; d) optic nerve glioma; e) 2 or more Lisch nodules (iris hamartomas) detected by slit-lamp examination, or 2 or more choroidal abnormalities detected by optical coherence tomography/near-infrared imaging; f) characteristic bony lesion, such as sphenoid dysplasia, anterolateral bowing of the tibia, or long bone pseudarthrosis; g) having a pathogenic heterozygous *NF1* variant with an allele variant fraction of 50% in normal tissues (e.g., leukocytes); h) one parent diagnosed with neurofibromatosis type I.

In some embodiments, the patient with neurofibromatosis type I meets at least 1 of the following criteria:
i) positive *NF1* germline mutation confirmed by genetic testing;
ii) the fulfillment of 2 or more of the following criteria confirmed by clinical and imaging examinations:
   a) 6 or more café-au-lait macules with a diameter (greatest dimension) greater than 5 mm before puberty, or a diameter greater than 15 mm after puberty;
   b) axillary or inguinal freckling;
   c) 2 or more neurofibromas of any type, or 1 or more plexiform neurofibromas;
   d) optic nerve glioma;
   e) two or more Lisch nodules (iris hamartomas);
   f) characteristic bony lesion, such as sphenoid dysplasia, or long bone cortical dysplasia or thinning;
   g) a first-degree relative diagnosed with neurofibromatosis type I.

In some embodiments, the patient with the disease associated with neurofibromatosis type I has not received a treatment with a mitogen-activated protein kinase (MEK) inhibitor.

### Technical Effects

The compound of formula I or the pharmaceutically acceptable salt thereof, or the pharmaceutical composition thereof of the present application exhibits good safety and anti-tumor activity.

The treatment regimen of the present application yields good efficacy in the treatment of diseases associated with neurofibromatosis type I. It yields superior effects in at least one of the following aspects: survival efficacy evaluations such as overall survival (OS) and median survival time; tumor response efficacy evaluations such as disease-free survival (DFS), median DFS, progression-free survival (PFS), 1-year progression-free survival rate (PFS > 12 m), time to progression (TTP), objective response rate (ORR), disease control rate (DCR), and duration of response (DOR); as well as tolerability or safety assessment.

The treatment regimen of the present application can effectively treat patients to achieve the best efficacy of stable disease (SD), partial response (PR), or complete response (CR).

The treatment regimen of the present application can improve the patient's pain scores, quality of life, motor function, or disfiguring symptoms.

### Definitions and Description

Unless otherwise stated, the following terms used in the present application shall have the following meanings. A certain term, unless otherwise specifically defined, should not be considered uncertain or unclear, but interpreted according to its common meaning in the art. When referring to a trade name herein, it is intended to refer to its corresponding commercial product or its active ingredient.

The term "treat", "treating", or "treatment" usually refers to acquiring needed pharmacological effects and/or physiological effects. The effects partially or fully stabilize or cure the disease and/or a side effect of the disease, and can be therapeutic. As used herein, "treat", "treating", or "treatment" encompasses any treatment of a disease in a patient, including: (a) inhibiting a symptom of the disease, i.e., blocking the progression of the disease; or (b) mitigating a symptom of the disease, i.e., causing the regression of the disease or the symptom.

The term "effective amount" refers to an amount of the compound of the present application for (i) treating a specific disease, condition, or disorder; (ii) alleviating, relieving, or eliminating one or more symptoms of a specific disease, condition, or disorder, or (iii) delaying onset of one or more symptoms of a specific disease, condition, or disorder described herein. The amount of the active substance (e.g., the compound of the present application) constituting the "effective amount" may vary according to factors such as the disease state, age, sex, and weight of the individual, and the ability of the therapeutic agent or therapeutic agent combination to elicit a desired response in the individual. The effective amount may also be determined routinely by those skilled in the art in accordance with their knowledge and the content of the present disclosure.

The term "administer", "administration", or "administering" refers to physically introducing the composition comprising a therapeutic agent to an entity using any one of a variety of methods and delivery systems known to those skilled in the art. In certain embodiments, the administration may be performed once, multiple times, and/or over one or more extended periods of time.

Unless otherwise stated, the term "dose" as used herein refers to a dose administered to a patient without considering the weight or the body surface area (BSA) of the patient. For example, a 60 kg human and a 100 kg human will receive the same dose of active ingredient (e.g., 5.0 mg of compound of formula I).

The term "pharmaceutically acceptable" is used herein for those compounds, materials, compositions, and/or dosage forms that are, within the scope of sound medical judgment, suitable for use in contact with the tissues of human beings and animals without excessive toxicity, irritation, allergic response, or other problems or complications, and commensurate with a reasonable benefit/risk ratio.

The term "pharmaceutically acceptable salt" includes salts formed from basic radicals and free acids and salts formed from acidic radicals and free bases.

Herein, the terms "subject", "patient", "entity", and "individual" are used interchangeably. In some embodiments, the term "subject" or "patient" refers to a mammal. In some embodiments, the subject or patient is a mouse. In some embodiments, the subject or patient is a human.

The term "single dose" refers to the smallest unit of packaging containing a certain quantity of pharmaceutical product; for example, in a box of seven capsules, each capsule is a single dose; or a vial of injection is a single dose.

The term "multiple doses" consists of multiple single doses.

The term "pharmaceutical composition" refers to a mixture consisting of one or more of the active ingredients or pharmaceutical combinations thereof of the present application and a pharmaceutically acceptable excipient. The pharmaceutical composition is intended to facilitate the administration of the compound or the pharmaceutical combination thereof of the present application to a subject.

The term "pharmaceutically acceptable excipient" refers to those that do not have a significant irritating effect on an organism and do not impair the biological activity and properties of the active compound. Suitable excipients are well known to those skilled in the art, such as carbohydrate, wax, water-soluble and/or water-swellable polymers, hydrophilic or hydrophobic material, gelatin, oil, solvent, and water.

The pharmaceutical composition of the present application can be prepared by combining the compound of the present application with a suitable pharmaceutically acceptable excipient, and can be formulated, for example, into a solid formulation (e.g., granule, tablet, pill, capsule, etc.) or a liquid formulation (e.g., injection).

The pharmaceutical composition of the present application may be manufactured using methods well known in the art, such as conventional mixing, dissolving, granulating, dragee-making, levigating, emulsifying, and lyophilizing.

A solid oral composition can be prepared by conventional mixing, filling, or tableting. For example, the solid oral composition can be obtained by the following method: mixing the active compound with solid excipients, optionally grinding the resulting mixture, adding additional suitable excipients if desired, and then processing the mixture into granules to obtain the core parts of tablets or dragees. Suitable excipients include, but are not limited to: binders, diluents, disintegrants, lubricants, glidants, sweeteners, flavoring agents, or the like.

As used herein, unless otherwise stated, the terms "comprise", "comprises", "comprising", or equivalents thereof are open-ended statements and mean that elements, components, and steps that are not specified may be encompassed in addition to those listed.

Unless otherwise specified, terms in the singular shall be deemed to include the plural and vice versa.

All patents, patent applications, and other identified publications are explicitly incorporated herein by reference for the purpose of description and disclosure. These publications are provided solely because they were disclosed prior to the filing date of the present application. All statements as to the dates of these documents or descriptions as to the contents of these documents are based on the information available to the applicant and do not constitute any admission as to the correctness of the dates or the contents of these documents. Moreover, in any country or region, any reference to these publications herein shall not be construed as an admission that the publications form part of the commonly recognized knowledge in the art.

### SPECIFIC EMBODIMENTS

For clarity, the present application is further described with the following examples, which are, however, not intended to limit the scope of the present application. All reagents used in the present application are commercially available and can be used without further purification. Reference can be made to WO2012059041 for the preparation of the compound of formula I in the examples.

### Example 1. Clinical Trial

### 1.1 Main inclusion criteria

(1) No gender limitation, aged 18-75 years;
(2) ECOG PS score ≤ 2, expected survival time ≥ 12 weeks for a patient with malignant peripheral nerve sheath tumor;
(3) patients with neurofibromatosis type I (including patients with malignant peripheral nerve sheath tumor) who were judged by the investigator as having the disease that cannot be completely surgically resected, requires systemic therapy, and has measurable lesions;
(4) having at least one evaluable lesion, with a lesion diameter of >3 cm (except for cutaneous type) and the lesion being visible in three consecutive sections;
(5) good major organ functions;
(6) patients enrolled in the cohort expansion phase required pathological confirmation for enrollment (e.g., patients with cutaneous neurofibromatosis type I, patients with plexiform neurofibromatosis type I, or patients with malignant peripheral nerve sheath tumor).

### 1.2 Administration regimen

Test drug: the capsule of the compound of formula I, which can be prepared with reference to WO2016188472. Administration method: oral administration under fasting conditions, once every day, continuous administration for 28 days constituted one cycle, until study termination criteria were met.

Administration dose: 5 mg-200 mg (e.g., 5 mg, 10 mg, 50 mg, 70 mg, or 100 mg) each time.

### 1.3 Evaluation criteria

Safety evaluation: The severity of adverse events was evaluated using the CTCAE 5.0 criteria;
efficacy evaluation: patients with cutaneous neurofibromatosis type I (cNF) were evaluated by direct measurement combined with investigator evaluation based on the natural history of the disease; patients with plexiform neurofibromatosis type I (PN) were evaluated according to the REiNS criteria; patients with malignant peripheral nerve sheath tumor (MPNST) were evaluated according to the RECIST 1.1 criteria.

### 1.4 Safety assessment

Incidence of adverse reactions: occurrence of all adverse events (AEs), serious adverse events (SAEs), and treatment-emergent adverse events (TEAEs).

### 1.5 Efficacy assessment

Objective response rate (ORR): refers to the percentage of subjects who achieved a complete response (CR) or partial response (PR) as determined by the investigator based on direct measurement results or RECIST 1.1; progression-free survival (PFS): refers to the time from the first administration to the occurrence of objective disease progression or recurrence, or death from any cause (whichever occurred first);
duration of response (DOR): defined as the time from the date of first documented tumor response to the date of first documented disease progression or the date of death from any cause (whichever occurred first) for subjects whose best response was complete response (CR) or partial response (PR);
disease control rate (DCR): the proportion of subjects whose tumor shrank or remained stable for a certain period, including cases of CR, PR, and stable disease (SD);
1-year progression-free survival rate (PFS > 12 m): the proportion of subjects who were free of disease progression and alive at 12 months;
overall survival (OS): refers to the time from the first administration to death from any cause;
impact on subject pain;
impact on subject's health-related quality of life (HRQoL);
impact on subject-related symptoms.

### 1.6 Trial results

**Table 1. Efficacy data for some patients**

| Patient | Type | Dose per administration | Administration cycle | Best efficacy |
|---|---|---|---|---|
| 1 | Plexiform | 5.0 mg | ≥C36 | At least PR |
| 2 | Cutaneous | 10.0 mg | ≥C36 | At least PR |
| 3 | Plexiform | 15.0 mg | ≥C24 | At least PR |
| 4 | Cutaneous | 15.0 mg | ≥C24 | At least PR |
| 5 | Cutaneous | 50.0 mg | ≥C12 | At least PR |
| 6 | Cutaneous | 50.0 mg | ≥C24 | At least PR |
| 7 | Cutaneous | 50.0 mg | ≥C12 | At least PR |
| 8 | Plexiform | 50.0 mg | ≥C18 | At least PR |
| 9 | Plexiform | 50.0 mg | ≥C6 | At least PR |
| 10 | Plexiform | 50.0 mg | ≥C6 | At least PR |
| 11 | Plexiform | 50.0 mg | ≥C6 | At least PR |
| 12 | Plexiform | 70.0 mg | ≥C6 | At least PR |
| 13 | Plexiform | 100.0 mg | ≥C18 | At least PR |

Conclusion: When the compound of formula I or the pharmaceutically acceptable salt thereof, or the pharmaceutical composition thereof was used to treat adult patients with neurofibromatosis type I, effective and sustained effects of tumor reduction could be achieved, demonstrating PFS benefit compared to patients under a natural disease course. For example, for patients with plexiform neurofibroma, the ORR was ≥20%, preferably ≥30%, and most preferably ≥40%; for patients with cutaneous neurofibroma, the ORR was ≥50%, preferably ≥70%, and most preferably ≥83%.

Partial safety data: The main adverse reactions were skin-related; the incidence of grade 3 or higher adverse reactions was low.

## Claims

1. Use of a compound of formula I or a pharmaceutically acceptable salt thereof, or a pharmaceutical composition of the compound of formula I or the pharmaceutically acceptable salt thereof for preparing a medicament for treating a disease associated with neurofibromatosis type I,

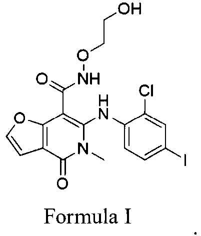

2. The use according to claim 1, wherein the compound of formula I or the pharmaceutically acceptable salt thereof, or the pharmaceutical composition thereof can be administered at a frequency of thrice every day, twice every day, once every day, once every two days, once every three days, once every four days, once every five days, once every six days, thrice every week, twice every week, once every week, once every two weeks, or once every three weeks.

3. The use according to claim 1, wherein a daily dose of the compound of formula I or the pharmaceutically acceptable salt thereof, or the pharmaceutical composition thereof is 0.1 mg-1000.0 mg, preferably 0.5 mg-500.0 mg, and more preferably 5.0 mg-200.0 mg.

4. The use according to claim 1, wherein the compound of formula I or the pharmaceutically acceptable salt thereof, or the pharmaceutical composition thereof is the compound of formula I or the pharmaceutically acceptable salt thereof, or the pharmaceutical composition thereof in a single dose of 0.1 mg-1000.0 mg, 0.5 mg-500.0 mg, 2.0 mg-250.0 mg, 5.0 mg-100.0 mg, or 5.0 mg-50.0 mg.

5. The use according to claim 1, wherein the compound of formula I or the pharmaceutically acceptable salt thereof, or the pharmaceutical composition thereof is administered orally.

6. The use according to claim 1, wherein the pharmaceutical composition of the compound of formula I or the pharmaceutically acceptable salt thereof is selected from the group consisting of a granule, a tablet, a pill, and a capsule, preferably selected from the group consisting of a tablet and a capsule, and more preferably selected from a capsule.

7. The use according to claim 1, wherein the disease associated with neurofibromatosis type I is selected from the group consisting of neurofibromatosis type I and malignant peripheral nerve sheath tumor.

8. The use according to claim 7, wherein the neurofibromatosis type I is selected from the group consisting of central, visceral, cutaneous, and peripheral neurofibromatosis type I.

9. The use according to claim 7, wherein the neurofibromatosis type I is selected from the group consisting of cutaneous neurofibroma, nodular neurofibroma, and plexiform neurofibroma.

10. The use according to claim 1, wherein the disease associated with neurofibromatosis type I is selected from the group consisting of cutaneous neurofibroma, plexiform neurofibroma, and peripheral nerve sheath tumor.
